# EUROPÄISCHE PATENTSCHRIFT

(11) **EP 1 641 769 B1**
(45) Veröffentlichungstag und Bekanntmachung des Hinweises auf die Patenterteilung: **20.09.2006**
(21) Anmeldenummer: 04735561.5
(22) Anmeldetag: 01.06.2004
(51) Int. Cl.: C07D 251/60

(54) **VERFAHREN ZUR HERSTELLUNG VON MELAMIN IM EINPHASIGEN ROHRREAKTOR**
METHOD FOR PRODUCING MELAMINE IN A SINGLE-PHASE TUBULAR REACTOR
PROCEDE DE PRODUCTION DE MELAMINE DANS UN REACTEUR TUBULAIRE MONOPHASE

(30) Priorität: 12.06.2003 DE 10326827
(43) Veröffentlichungstag der Anmeldung: 05.04.2006
(73) Patentinhaber: AMI Agrolinz Melamine International GmbH, 4021 Linz (AT)
(72) Erfinder: SCHRÖDER, Frank, 04683 Naunhof (DE); FELLNER, Johannes, A-4400 Steyr (AT); BUCKA, Hartmut, A-4622 Eggendorf (AT)
(74) Vertreter: Gross, Felix
(86) Internationale Anmeldenummer: PCT/EP2004/005882
(87) Internationale Veröffentlichungsnummer: WO 2004/111016

(56) Entgegenhaltungen:
- WO-A-02/12206
- WO-A-02/34730
- WO-A-97/34879
- WO-A-99/00374

## Beschreibung

Die Erfindung betrifft ein Verfahren nach dem Oberbegriff des Anspruchs 1.

Jeder Reinstoff weist als charakteristische Stoffeigenschaften eine kritische Temperatur, einen kritischen Druck und ein kritisches Volumen auf. Befindet sich ein Stoff im überkritischen Zustand, so existiert keine Trennung mehr zwischen einer Flüssigphase und einer Gasphase. Das überkritische Fluid verhält sich in völlig eigner Weise. Zum Beispiel kann ein überkritischer Stoff eine Viskosität eines Gases aufweisen bei der Dichte einer Flüssigkeit. Durch die Variation des Drucks und der Temperaturbedingungen können die Fließeigenschaften eines Stoffes meist in weiten Bereichen gezielt beeinflusst werden.

Bei einer Mischung von überkritischen Stoffen (z.B. Ammoniak und Kohlendioxid) mit unterkritischen Stoffen (je nach Temperatur z.B. Melamin) kann es bei vollständiger Mischbarkeit zu Gemischeigenschaften kommen, die denen überkritischer Stoffe entsprechen. Dies bedeutet, dass das Gemisch einphasig vorliegt.

Es ist bekannt (siehe z.B. Ullmann's Encyclopedia of Industrial Chemistry, 5. Auflage, Vol. A16, S. 171 ff), Melamin durch thermische Umsetzung zu Melamin gemäß 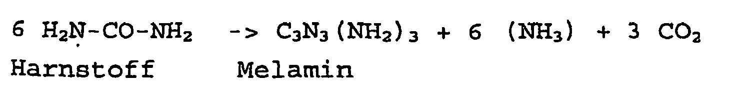 herzustellen. Dabei wird geschmolzener Harnstoff (T_{schmelz} = 132 °C) eingesetzt. Als Reaktionsprodukt fallen Melamin, Ammoniak und Kohlendioxid an. Ammoniak und Kohlendioxid werden zusammen als Offgas bezeichnet.

Grundsätzlich gibt es zwei Herstellungsverfahren für Melamin, nämlich nicht-katalytische, Hochdruckverfahren und katalytische Niederdruckverfahren.

Bei Niederdruckverfahren wird eine Gasphasenreaktion bei Drücken von typischerweise 10 bar und Temperaturen zwischen 390 und 410 °C durchgeführt. Bei den Hochdruckverfahren wird eine Flüssigphasenreaktion bei Temperaturen zwischen 370 bis 425 °C und Drücken zwischen 70 und 150 bar durchgeführt.

Hochdruckverfahren zur Melaminherstellung sind etwa aus WO99/00374, WO97/34879, WO02/12206 oder WO02/34730 bekannt.

Dabei besteht bei beiden Verfahrensvarianten der Nachteil, dass große Mengen an Offgas anfallen. Da diese Stoffe bei den Reaktionsbedingungen in der Gasphase vorliegen, muss insbesondere der Reaktor entsprechend groß dimensioniert werden. Da das Reaktionsgemisch stark korrosiv ist, müssen die relativ großen Reaktionsbehälter aus besonderem Material, z.B. Titan gefertigt werden.

Eine Verwendung eines kompakten Rohrreaktors kommt bei diesen Bedingungen nicht in Betracht, da das Offgas den nicht umgesetzten Harnstoff sehr schnell aus dem Reaktor schiebt. Es läge ein zweiphasiges Reaktionsgemisch vor. Außerdem ist der Wärmeübergang unter diesen Bedingungen schlecht, so dass sehr große Wärmeübergangsflächen benötigt werden, um die hohe Reaktionswärme einzubringen, was die Reaktoren weiterhin stark verkompliziert.

Der vorliegenden Erfindung liegt die Aufgabe zugrunde, ein Verfahren zu schaffen, mit denen eine effizientere Reaktion zu Melamin möglich ist.

Diese Aufgabe wird erfindungsgemäß durch ein Verfahren mit den Merkmalen des Anspruchs 1 gelöst.

Erfindungsgemäß läuft die Reaktion von Harnstoff zu Melamin zumindest teilweise bei Reaktionsbedingungen ab, bei denen mindestens ein Edukt, Zwischenprodukt und / oder Endprodukt in einem überkritischen Zustand vorliegt. Auch bildet die Mischung aus mindestens einem Edukt, Zwischenprodukt und / oder Endprodukt eine im Wesentlichen homogenen Phase, insbesondere liegen alle Edukte, Zwischenprodukte und / oder Endprodukte in vollständiger Lösung vor.

Durch die Verwendung eines "überkritischen Gemisches" lässt sich eine Einphasigkeit herstellen, durch die das Reaktorvolumen stark reduziert werden kann.

Dadurch kann Material und Bauraum eingespart werden. Dies führt auch zu einem verbesserten Sicherheitsaspekt, da die unter Hochdruck stehende Stoffmenge kleiner ist. Durch den hohen Druck wird im Übrigen auch die Melaminqualität verbessert, da der Anteil an Nebenprodukten verringert wird. Die überkritischen Stoffeigenschaften führen bei entsprechender Strömungsgeschwindigkeit, die wegen dieser Stoffeigenschaften bei erträglichem Druckverlust erreichbar ist, zu einer sehr hohen Wärmeübergangszahl, die den Eintrag der notwendigen Reaktionsenergie über die relativ kleine Rohrwandfläche gestattet.

Vorteilhaft ist es, wenn die Reaktion zumindest teilweise bei einem Druck über 550 bar, insbesondere zwischen 600 und 800 bar abläuft. In vorteilhafter Weise läuft die Reaktion zumindest teilweise bei einer Temperatur von mindestens 350 °C, insbesondere 400 °C ab.

In einer vorteilhaften Ausgestaltung des erfindungsgemäßen Verfahrens läuft die Reaktion in einem kontinuierlichen Rohrreaktor ab. Dabei ist es besonders vorteilhaft, wenn der Rohrreaktor zur Erzeugung der Reaktionstemperatur wenigstens teilweise beheizt wird.

Ferner ist es vorteilhaft, wenn flüssiger Harnstoff als Edukt eingesetzt wird.

Das Erreichen des Reaktionsdrucks wird vorteilhafterweise vor dem Reaktor durch eine Hochdruckpumpe bewirkt, die den flüssigen Harnstoff fördert.

In einer weiteren vorteilhaften Ausgestaltung des erfindungsgemäßen Verfahrens wird das Reaktionsprodukt des Reaktors zur Verfestigung des Melamins in einen Entspannungsbehälter mit Druck kleiner als 200 bar, insbesondere Atmosphärendruck entspannt.

Durch die Entspannung von einem sehr hohen Druck tritt eine deutliche Abkühlung ein. Vorteilhafterweise ist der , Entspannungsbehälter temperierbar.

Dabei kann es vorteilhaft sein , wenn ein im Entspannungsbehälter entstehendes Offgas, mindestens den Druck einer Harnstoffsynthese aufweist, so dass es einer Harnstoffsyntheseanlage zugeführt werden kann.

Auch ist es vorteilhaft, wenn die erfindungsgemäße Vorrichtung eine Entspannungsvorrichtung, insbesondere ein Ventil zur kontrollierten Entspannung in den Entspannungsbehälter aufweist.

Des Weiteren ist es vorteilhaft, wenn eine Regelungsvorrichtung zur Druckregelung im Reaktor vorgesehen ist, besonderes wenn die Regelungsvorrichtung für den Reaktordruck mit der Entspannungsvorrichtung gekoppelt ist.

Verwendung findet eine Vorrichtung, wobei ein Reaktor als Rohrreaktor für überkritische Reaktionsbedingungen ausgebildet ist. Ein solcher Reaktor kann sehr kompakt gebaut werden.

In vorteilhafter Weise weist der Rohrreaktor eine TitanLegierung auf, um gegen korrosive Medien geschützt zu sein.

Auch ist es vorteilhaft, wenn die Vorrichtung eine Entspannungsvorrichtung zur Entspannung von Reaktionsprodukten in einen Entspannungsbehälter aufweist.

Die Erfindung wird nachfolgend unter Bezugnahme auf die Figuren der Zeichnungen an einem Ausführungsbeispiel näher erläutert. Es zeigen:
- Fig. 1: ein Verfahrensfließbild für einen überkritischen Melaminprozess;
- Fig. 2a-2d: Messergebnisse für die Änderung des Volumens in Abhängigkeit vom Druck.

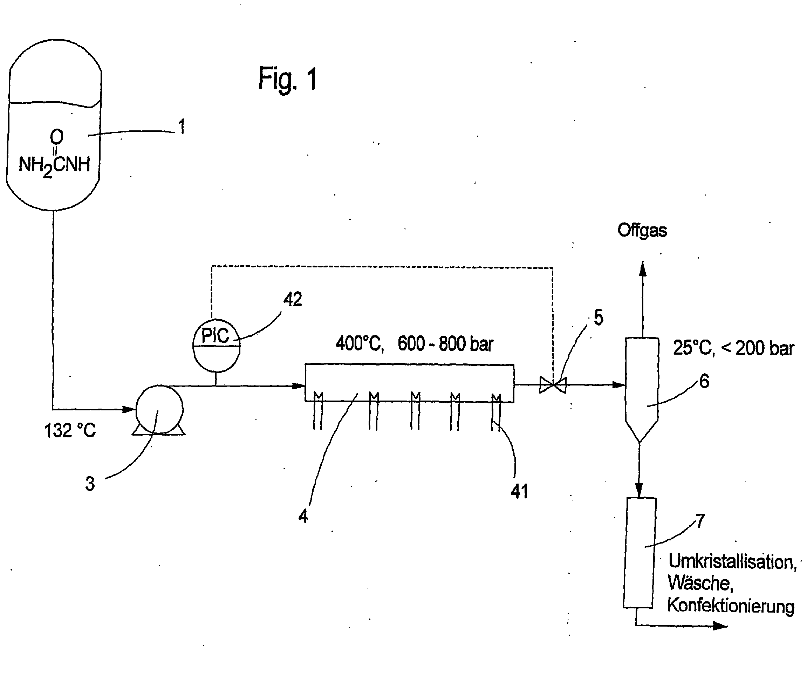

In Fig. 1 ist eine Ausführungsform des erfindungsgemäßen Verfahrens dargestellt. Dabei geht es um die Produktion von Melamin (2,4,6-Triamino-1,3,5,-Triazin, C₃N₆H₆) aus Harnstoff (Carbamid, Co(NH₂)₂).

Dazu wird Harnstoff in einem Vorlagebehälter 1 vorgelegt und nach Bedarf einem Reaktor 4 zugeführt.

Der Harnstoff im Vorlagebehälter 1 wird bei einer Temperatur von etwas mehr als 132°C vorgelegt. Anschließend wird die Harnstoffschmelze durch eine Hochdruckpumpe 3 (z.B. Membran-Kolbenpumpe oder Zahnradpumpe) auf ein Druckniveau gebracht, das so hoch liegt, dass die nachfolgende Reaktion im Reaktor 4 im Wesentlichen einphasig verlaufen kann.

Das Phasenverhalten kann bestimmt werden, indem die Volumenänderung in Abhängigkeit der Temperatur bei konstantem Druck gemessen wird. Bei niedrigen, konstant gehaltenen Drücken (z.B. 130 bar), d.h. beim Vorliegen eines zweiphasigen Gemisches, ist ein sprunghafter Anstieg des Volumens bei steigender Temperatur zu erkennen; eine gasförmige Phase entsteht. Führt man die gleiche Messung bei einem höheren Druck (z.B. 800 bar) durch, so erkennt man, dass die Volumenzunahme mit steigender Temperatur ohne Sprung ansteigt; es entsteht keine neue Phase. Zusätzlich kann man die für die Erwärmung notwendige Wärmemenge im Kalorimeter bestimmen.

Der Druck im Reaktor 4 wird, um Einphasigkeit zu erreichen, über 550 bar, vorzugsweise im Bereich von 600 bis 800 bar liegen.

Zur Erzielung eines akzeptablen Umsatzes wird der Reaktor 4 auf mindestens 350 °C, vorzugsweise um 400°C geheizt. Dazu wird der Reaktor 4 über seine Länge mit einer Heizung 41 auf die Prozesetemperatur gebracht. Bei diesen Bedingungen liegt das Reaktionsgemisch in einer einzigen Phase vor. Die Strömung wird in diesem Fall als Pfropfenströmung ausgebildet, so dass sich ein intensiver Wärmeübergang einstellen kann.

Die ansonsten als zweite Phase vorliegenden Offgase sind Teil der Reaktionsmasse mit überkritischen Stoffeigenschaften. Der Reaktor 4 ist als kontinuierlicher Rohrreaktor ausgebildet.

Nach dem Reaktor 4 werden die Reaktionsprodukte (Melamin, Ammoniak, Kohlendioxid, NH₂COONH₄, NH₂CONH₂, Reste von Harnstoff (z.B. 15%)) über eine Entspannungsvorrichtung 5 in einen Entspannungsbehälter 6 geleitet.

Die Entspannungsvorrichtung 5 ist als Ventil ausgebildet. Durch die Entspannung der Reaktionsprodukte bildet sich im Entspannungsbehälter 6 ein Druck aus. Es kann ein geringer Druck gewählt werden um die Anlagenkosten gering zu halten, es kann aber auch ein Druck von etwa 200 bar gewählt werden, um das Offgas in eine Harnstoffanlage zurück zu führen. Auch Quenschen in z.B. Ammoniak oder Wasser ist denkbar.

Die Temperatur im Entspannungsbehälter 6 kann zwischen Raumtemperatur und der Hydrolyse- bzw. Zersetzungstemperatur des Melamins gewählt werden. Dabei verfestigt sich das Melamin sehr schnell oder kann im Quenchmedium gelöst werden. Der Entspannungsbehälter 6 ist mit einer Temperiervorrichtung ausgestattet, die wahlweise ein Heizen oder Kühlen erlaubt. Durch die Temperierung kann z.B. der physikalische Zustand des Melamins in weiten Grenzen beeinflusst werden.

Das Ventil 5 dient auch.als Stellglied für eine Regelungsvorrichtung 42, die hier als PI-Regler ausgebildet ist. Grundsätzlich können natürlich auch andere Regelmechanismen, z.B. Mehrgrößenregelungen gewählt werden. Auch sind u.U. notwendige Sicherheitsventile oder Sicherheitsregelungen hier nicht dargestellt.

Nach dem Entspannen werden die gasförmigen Offgase oben aus dem Entspannungsbehälter 6 abgeführt. Wenn der Entspannungsbehälter 6 entsprechend temperiert ist, kann das Carbamat (NH₂COONH₄) in Kohlendioxid und Ammoniak (Offgas) thermisch zersetzt werden. Bei einem geeigneten Druckniveau im Entspannungsbehälter 6 kann das Offgas in den Hochdruckteil einer Harnstoffsynthese rückgeführt werden.

Die festen oder im Quenchmedium aufgenommenen Bestandteile (Melamin, NH₂COONH₄, NH₂CONH₂) werden aus dem Entspannungsbehälter 6 ausgetragen und werden dann zu einem weiteren Verarbeitungsschritt 7, z.B. einer Konfektionierung oder zu einer hier nicht näher dargestellten Umkristallisation zu einer Wäsche geführt.

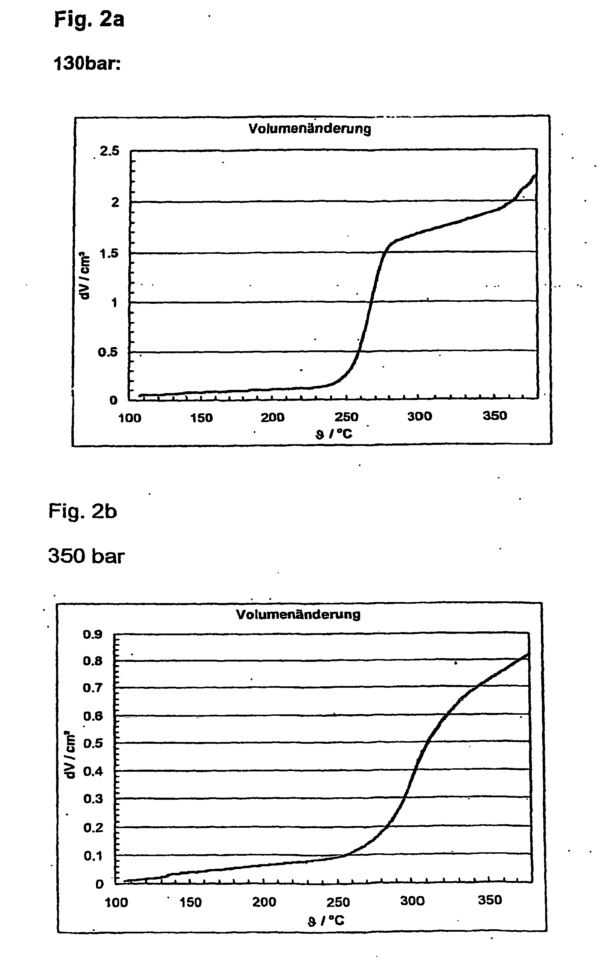

In Fig. 2a bis 2d sind Messergebnisses dargestellt, die bei der Herstellung von Melamin in Gegenwart von Ammoniak und Kohlendioxid gewonnen wurden. Dargestellt ist jeweils die Änderung des Volumens in Abhängigkeit von der Temperatur. Der Druck wurde jeweils konstant gehalten.

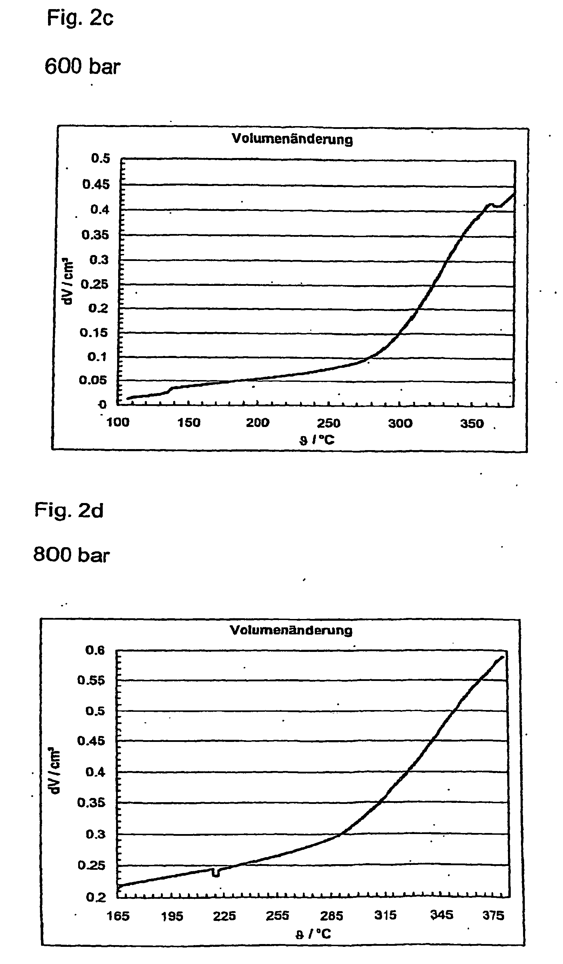

Bei dem niedrigsten Druck von 130 bar (Fig. 2a) ist erkennbar, dass oberhalb von ca. 265°C eine starke Volumenzunahme einsetzt, d.h. es entsteht eine zweite, gasförmige Phase. Bei der nächsten Druckstufe von 350 bar (Fig. 2b) fällt die Druckzunahme oberhalb von 300 °C nicht mehr ganz so heftig auf; der Gradient ist geringer. Dieser Trend setzt sich bei 600 bar (Fig. 2c) fort. Eine Verdampfung dürfte im Bereich von 300 bis 350 °C stattfinden.

Fig. 2d zeigt schließlich die Volumenzunahme bei einem Druck von 800 bar. Eine abrupte Volumenzunahme ist praktisch nicht erkennbar. Dies zeigt, dass die Mischung aus Edukten, Zwischenprodukten und Endprodukten eine im Wesentlichen homogene Mischung bilden, deren Volumen relativ monoton mit der Temperatur wächst.

Die Erfindung beschränkt sich in ihrer Ausführung nicht auf die vorstehend angegebenen bevorzugten Ausführungsbeispiele. Vielmehr ist eine Anzahl von Varianten denkbar, die von dem erfindungsgemäßen Verfahren und der erfindungsgemäßen Vorrichtung auch bei grundsätzlich anders gearteten Ausführungen Gebrauch machen.

### Bezugszeichenliste

- 1: Vorlagebehälter
- 3: Hochdruckpumpe
- 4: Reaktor
- 5: Entspannungsvorrichtung
- 6: Entspannungsbehälter mit Temperiereinrichtung
- 7: Weiterverarbeitungsschritt

## Patentansprüche

1. Verfahren zur Herstellung von Melamin durch thermische Umwandlung von Harnstoff
**dadurch gekennzeichnet**,
a) dass die Reaktion von Harnstoff zu Melamin zumindest teilweise bei Reaktionsbedingungen abläuft, bei denen mindestens ein Edukt, Zwischenprodukt und / oder Endprodukt in einem überkritischen Zustand vorliegt, und
b) die Mischung aus mindestens einem Edukt, Zwischenprodukt und / oder Endprodukt im Wesentlichen eine homogene Phase bilden, insbesondere alle Edukte, Zwischenprodukte und / oder Endprodukte in vollständiger Lösung vorliegen.

2. Verfahren nach Anspruch 1, **dadurch gekennzeichnet, dass** die Reaktion zumindest teilweise bei einem Druck über 550 bar, vorzugsweise zwischen 600 bar und 800 bar abläuft.

3. Verfahren nach Anspruch 1 oder 2, **dadurch gekennzeichnet, dass** die Reaktion zumindest teilweise bei einer Temperatur von mindestens 350 °C, insbesondere 400 °C abläuft.

4. Verfahren nach mindestens einem der vorhergehenden Ansprüche, **dadurch gekennzeichnet, dass** die Reaktion in einem kontinuierlichen Rohrreaktor (4) durchgeführt wird.

5. Verfahren nach Anspruch 4, **dadurch gekennzeichnet, dass** der Rohrreaktor (4) wenigstens teilweise beheizt wird.

6. Verfahren nach mindestens einem der vorhergehenden Ansprüche, **dadurch gekennzeichnet, dass** Harnstoff als flüssiges Edukt eingesetzt wird.

7. Verfahren nach mindestens einem der vorhergehen Ansprüche, **dadurch gekennzeichnet, dass** das Edukt vor dem Reaktor (4) durch eine Hochdruckpumpe (2) auf den notwendigen Reaktionsdruck gebracht wird.

8. Verfahren nach mindestens einem der vorhergehenden Ansprüche, **dadurch gekennzeichnet, dass** das Reaktionsprodukt des Reaktors (4) zur Verfestigung des Melamins in einen Entspannungsbehälter (6) mit einem Druck unterhalb von 200 bar, insbesondere Atmosphärendruck entspannt wird.

9. Verfahren nach mindestens einem der vorhergehenden Ansprüche, **dadurch gekennzeichnet, dass** ein im Entspannungsbehälter (6) entstehendes Offgas, mindestens den Druck einer Harnstoffsynthese aufweist, so dass es einer Harnstoffsyntheseanlage zugeführt werden kann.

10. Verfahren nach Anspruch 9, **dadurch gekennzeichnet, dass** der Entspannungsbehälter (6) temperiert ist.

11. Verfahren nach Anspruch 10, **gekennzeichnet durch** eine Entspannungsvorrichtung (5), insbesondere ein Ventil zur kontrollierten Entspannung in den Entspannungsbehälter (6).

12. Verfahren nach mindestens einem der vorhergehenden Ansprüche, **gekennzeichnet durch** eine Regelungsvorrichtung (5) zur Druckregelung im Reaktor (4).

13. Verfahren nach Anspruch 10, **dadurch gekennzeichnet, dass** die Regelungsvorrichtung (5) für den Reaktordruck mit der Entspannungsvorrichtung (3) gekoppelt ist.

## Claims

1. Process for preparing melamine by thermally converting urea,
**characterized in that**
a) the reaction of urea to give melamine proceeds at least partly under reaction conditions under which at least one reactant, intermediate and/or end product is in a supercritical state, and
b) the mixture of at least one reactant, intermediate and/or end product forms a substantially homogeneous phase, in particular all reactants, intermediates and/or end products are fully dissolved.

2. Process according to Claim 1, **characterized in that** the reaction proceeds at least partly at a pressure above 550 bar, preferably between 600 bar and 800 bar.

3. Process according to Claim 1 or 2, **characterized in that** the reaction proceeds at least partly at a temperature of at least 350°C, in particular 400°C.

4. Process according to at least one of the preceding claims, **characterized in that** the reaction is carried out in a continuous tubular reactor (4).

5. Process according to Claim 4, **characterized in that** the tubular reactor (4) is at least partly heated.

6. Process according to at least one of the preceding claims, **characterized in that** urea is used as a liquid reactant.

7. Process according to at least one of the preceding claims, **characterized in that** the reactant is brought to the required reaction pressure upstream of the reactor (4) by a high-pressure pump (2).

8. Process according to at least one of the preceding claims, **characterized in that** the reaction product of the reactor (4) is decompressed, to solidify the melamine, into a decompression vessel (6) having a pressure below 200 bar, in particular atmospheric pressure.

9. Process according to at least one of the preceding claims, **characterized in that** an offgas formed in the decompression vessel (6) has at least the pressure of a urea synthesis, so that it can be fed to a urea synthesis plant.

10. Process according to Claim 9, **characterized in that** the decompression vessel (6) is heated.

11. Process according to Claim 10, **characterized by** a decompression apparatus (5), especially a valve for controlled decompression into the decompression vessel (6).

12. Process according to at least one of the preceding claims, **characterized by** a regulation apparatus (5) for pressure regulation in the reactor (4).

13. Process according to Claim 10, **characterized in that** the regulation apparatus (5) for the reactor pressure is coupled to the decompression apparatus (3).

## Revendications

1. Procédé en vue de la fabrication de mélamine par conversion thermique d'urée, **caractérisé en ce que**
a) la réaction de l'urée pour former de la mélamine se déroule tout au moins partiellement sous des conditions de réaction lors desquelles un produit de départ, un produit intermédiaire et/ou un produit final est présent dans un état sur-critique, et **en ce que**
b) le mélange d'au moins un produit de départ, un produit intermédiaire et/ou un produit final forme pour l'essentiel une phase homogène, en particulier tous les produits de départ, tous les produits intermédiaires et/ou tous les produits finaux étant présents en solution complète.

2. Procédé selon la revendication 1, **caractérisé en ce que** la réaction se déroule tout au moins en partie à une pression au-delà de 550 bars, de préférence comprise entre 600 bars et 800 bars.

3. Procédé selon la revendication 1 ou 2, **caractérisé en ce que** la réaction se déroule tout au moins en partie à une température d'au moins 350°C, en particulier de 400°C.

4. Procédé selon au moins l'une quelconque des revendications précédentes, **caractérisé en ce que** la réaction est effectuée dans un réacteur tubulaire (4) en mode continu.

5. Procédé selon la revendication 4, **caractérisé en ce que** le réacteur tubulaire (4) est au moins partiellement chauffé.

6. Procédé selon au moins l'une quelconque des revendications précédentes, **caractérisé en ce que** l'urée est utilisée en tant que produit de départ liquide.

7. Procédé selon au moins l'une quelconque des revendications précédentes, **caractérisé en ce que** le produit de départ est amené, avant le réacteur (4), grâce à une pompe haute pression (2), à la pression de réaction nécessaire.

8. Procédé selon au moins l'une quelconque des revendications précédentes, **caractérisé en ce que** le produit de réaction du réacteur (4) est détendu en vue de la solidification de la mélamine dans un récipient de détente (6) avec une pression en dessous de 200 bars, en particulier à la pression atmosphérique.

9. Procédé selon au moins l'une quelconque des revendications précédentes, **caractérisé en ce qu'**un gaz de rejet, se formant dans le récipient de détente (6), présente au moins la pression d'un synthèse de l'urée, de telle sorte qu'il peut être acheminé à une installation de synthèse de l'urée.

10. Procédé selon la revendication 9, **caractérisé en ce que** le récipient de détente (6) est tempéré.

11. Procédé selon la revendication 10, **caractérisé par** un dispositif de détente (5), en particulier par une vanne en vue de la détente contrôlée dans le récipient de détente (6).

12. Procédé selon au moins l'une quelconque des revendications précédentes, **caractérisé par** un dispositif de régulation (5) en vue de la régulation de pression dans le réacteur (4).

13. Procédé selon la revendication 10, **caractérisé en ce que** le dispositif de régulation (5) pour la pression du réacteur est couplé au dispositif de détente (3).
